# EUROPÄISCHE PATENTSCHRIFT

(11) **EP 0 519 901 B1**
(45) Veröffentlichungstag und Bekanntmachung des Hinweises auf die Patenterteilung: **18.08.1999**
(21) Anmeldenummer: 92890145.3
(22) Anmeldetag: 16.06.1992
(51) Int. Cl.: A61K 35/16, A61L 2/04, A61L 2/00

(54) **Durch Hitze und/oder Detergens virusinaktiviertes Blutprodukt**
Virus-inactivated blood product prepared by treatment with heat and/or detergent
Produit sanguin dans lequel les virus ont été inactivés au moyen de chaleur et/ou détergent

(30) Priorität: 20.06.1991 AT 123791
(43) Veröffentlichungstag der Anmeldung: 23.12.1992
(60) Teilanmeldung: 98111667.6
(73) Patentinhaber: IMMUNO Aktiengesellschaft, A-1221 Wien (AT)
(72) Erfinder: Eibl, Johann, Dr., A-1180 Wien (AT); Elsinger, Friedrich, Dr., A-1130 Wien (AT); Linnau, Yendra, Dr., A-1224 Wien (AT); Wöber, Günter, Prof. Dr., A-2522 Oberwaltersdorf (NÖ) (AT)
(74) Vertreter: Sonn, Helmut

(56) Entgegenhaltungen:
- EP-A- 0 050 061
- EP-A- 0 099 445
- EP-A- 0 124 044
- EP-A- 0 324 729
- EP-A- 0 341 103
- EP-A- 0 378 208
- EP-B- 0 131 740
- WO-A-82/03871
- CHEMICAL ABSTRACTS, vol. 84, no. 16, 19. April 1976, Columbus, Ohio, US; abstract no. 111640n, FUNAKOSHI SATOSHI 'HOMOGENEOUS HEPATITIS B ANTIGEN.' Seite 405 ; & JP-A-50 160 420 (GREEN CROSS CORP) 25 Dezember 1975
- Eur. J. Epidemiol. 3, 103-118 (1987)
- The Lancet 339, 819 (1992)
- Commission of the European Communities, "Ad Hoc Working party On Biotechnology/Pharmacy 111/8115/89-EN Final, pp. 1-15
- Biologicals 19, 151-159 (1991)
- Develop. biol. Standard. 75, 227-232 (1991)
- Curr. Stud. Hematol. Blood Transfus. 56, 23-33 (1989)
- The Yale Journal of Biology and Medicine 63, 361-369 (1990)

## Beschreibung

Die Erfindung betrifft ein Verfahren zur Herstellung eines virusinaktivierten Blutprodukts, sowie ein Verfahren zur Bestimmung der Virus-Inaktivierungskapazität von einer Inaktivierungsbehandlung.

Unter Blutprodukten werden Produkte aus menschlichem oder tierischem Blut bzw. Plasma verstanden, die zur therapeutischen, prophylaktischen oder diagnostischen Anwendung bestimmt sind. Solche Produkte können Enzyme, Proenzyme einschließlich Gerinnungsfaktoren, Enzymkofaktoren, Enzyminhibitoren, Immunglobuline, Albumin, Plasminogen, Fibrinogen, Fibronectin oder Plasma enthalten.

Die Verabreichung von Blutprodukten birgt ein Infektionsrisiko aufgrund der im Spenderplasma eventuell vorhandenen infektiösen Agentien, wie Hepatitis- oder AIDS-Viren. Auch wenn ausschließlich Plasma verarbeitet wird, welches auf Freiheit von diesen infektiösen Agentien getestet wurde, kann aufgrund der begrenzten Sensitivität der Testmethoden eine Infektionsgefahr beim Patienten nicht ausgeschlossen werden. Bei der Herstellung von Blutprodukten ist man daher gezwungen, durch verschiedene Maßnahmen eventuell vorhandene infektiöse Agentien zu inaktivieren.

Es ist eine umfangreiche Literatur vorhanden, die sich mit der Inaktivierung von Krankheitserregern in Blutprodukten befaßt.

Die verschiedenen Verfahren umfassen:
- Erhitzen der Blutprodukte in wässeriger Lösung, gegebenenfalls unter Zusatz viruzider Substanzen,
- Erhitzen der Blutprodukte in wässeriger Lösung in Anwesenheit von Stabilisatoren,
- Behandeln der Blutprodukte mit organischen Lösungsmitteln und/oder Detergentien,
- Erhitzen der Blutprodukte in trockenem und in nassem Zustand,
- Kombiniertes Behandeln der Blutprodukte mit einem organischen Lösungsmittel/Detergens und Erhitzen der Blutprodukte in trockenem Zustand.

Das Bestreben bei allen diesen Inaktivierungsverfahren geht dahin, die potentielle Infektivität der Präparationen aufzuheben, ihre biologische Aktivität aber weitgehend zu erhalten. Dieses Ziel konnte jedoch bisher nur bei Albuminpräparationen erreicht werden, u.zw. durch Erhitzen von wässerigen Albuminlösungen bei einer Temperatur von 60°C während 10 h, da Albumin wesentlich stabiler gegenüber Hitzeeinwirkung ist als alle anderen Blutproteine.

Im einzelnen sind zum Stand der Technik beispielsweise die folgenden Literaturstellen anzuführen:

Die DE-A - 29 16 711 beschreibt ein Verfahren zur Behandlung von Gerinnungsfaktoren enthaltenden Präparationen in wässeriger Lösung unter Anwendung einer Temperatur von 30 bis 100°C, wobei der Lösung der Gerinnungsfaktoren eine Aminosäure und ein Mono-, Oligosaccharid oder Zuckeralkohol zugesetzt werden.

Die EP-A2-0 053 338 beschreibt ein Verfahren zur Inaktivierung von Hepatitisviren in Faktor IX und X enthaltenden Präparationen, wobei eine Erwärmung der wässerigen Lösung eines Blutpräparates in Gegenwart von Calciumionen und gegebenenfalls einer Aminosäure und/oder eines Saccharids oder Zuckeralkohols bei Temperaturen von bis zu 100°C vorgenommen wird.

In der EP-A2-0 035 204 wird ein Verfahren zur Inaktivierung von wässerigen Proteinlösungen, die Faktor VIII, Fibronectin, Globulin, Fibrinogen und andere Proteine enthalten können, beschrieben, wobei die Komposition mit einem Polyol gemischt und die Mischung auf eine Temperatur von 60 bis 75°C erhitzt wird.

In der EP-A2-0 052 827 ist ein Verfahren zur Inaktivierung von Hepatitisviren in einer wässerigen, die Faktoren II und VII enthaltenden Lösung in Anwesenheit eines Chelatbildners und gegebenenfalls einer Aminosäure und/oder eines Saccharids oder Zuckeralkohols beschrieben.

In der US-A-4,379,085 ist ein Verfahren zur Hitzeinaktivierung eines Plasmaproteins, wie C₁-Inhibitor oder Faktor IX, in wässeriger Lösung in Gegenwart von Kalium- oder Ammoniumcitrat beschrieben.

In der EP-A2-0 077 870 ist ein Inaktivierungsverfahren beschrieben, bei welchem eine wässerige, Faktor VIII enthaltende Lösung mit Aminosäuren, Monosacchariden, Oligosacchariden, Zuckeralkoholen und Kohlenwasserstoff-oder Hydroxykohlenwasserstoffcarbonsäuren mit 3 bis 10 Kohlenstoffatomen auf eine Temperatur von 50 bis 80°C erhitzt wird.

In der PCT-Anmeldung WO 83/04371 ist ein Verfahren zum Inaktivieren von Hepatitisviren beschrieben, wobei eine das Virus enthaltende Präparation bei einer Temperatur von 4 bis 40°C mit einem Halogenkohlenwasserstoff, insbesondere Chloroform, behandelt wird.

Die EP-B1-0 015 055 beschreibt ein Verfahren zur Behandlung eines Blutproduktes, wobei das Produkt in wasserfreiem Zustand einer Mikrowellenbestrahlung ausgesetzt wird, um vorhandene Mikroorganismen zu inaktivieren.

Rosenberg et al. beschreiben in einer Abhandlung des XII. International Congress on Blood Transfusion, Abstracts, "MIR" Publishers, Moscow 1969, pp. 473-475 ein Verfahren zur Inaktivierung von albuminhaltigen Präparationen und Fibrinogen in trockenem Zustand durch 10stündiges Erhitzen auf 60°C.

Die EP-A2-0 094 611 beschreibt ein Verfahren zur Behandlung einer Faktor VIII enthaltenden Komposition im trockenen, z.B. lyophilisierten, Zustand, unter Anwendung einer Temperatur von wenigstens 60°C zwecks Inaktivierung von vorhandenen Hepatitisviren.

Die veröffentlichte PCT-Anmeldung WO 82/03871 beschreibt ein Verfahren zur Behandlung von Blutgerinnungsenzyme enthaltenden Zubereitungen, wobei diese zur Inaktivierung vorhandener infektiöser Viren im trockenen Zustand erhitzt werden; als trockener Zustand wird ein solcher mit weniger als 5 Gew.% (0,05) Wasser definiert.

Es hat sich gezeigt, daß die Methode der Trockenerhitzung von lyophilisiertem Faktor VIII-Konzentrat während 10 h bei 60°C zwar eine gewisse Virusinaktivierung bewirkt, daß jedoch durch Verabreichung der trockenerhitzten Produkte Hepatitis- und auch AIDS-Viren übertragen werden (Eur. J. Epidemiol. 3, 103-118 (1987)). Um den Wirkungsgrad der Trockenerhitzung zu steigern wird in der PCT-Anmeldung WO 88/08710 eine Folge von Hitzebehandlungen vorgeschlagen.

Ebenso werden in der EP-A-0 378 208 proteinhältige Kompositionen einer Behandlung mit Trialkylphosphat in Kombination mit einer Trockenhitzebehandlung unterworfen.

Das Verfahren der EP-B - 0 159 311 sieht eine Behandlung von Blutprodukten im festen, nassen Zustand vor. Es wird ein Gehalt an Wasser, Methanol oder Äthanol von mehr als 0,05 (5 Gew.%) und weniger als 0,70 (70 Gew.%) eingestellt und in einem geschlossenen Behälter bei einer Temperatur im Bereich von 50 bis 121°C erhitzt.

In der EP-B - 0 050 061 ist ein Verfahren beschrieben, welches die Behandlung biologischer und pharmazeutischer Produkte mit 0,25 bis 10 Gew.% eines nicht denaturierenden Amphiphils (Detergens) umfaßt. In der EP-B - 0 131 740 wird jedoch gezeigt, daß die Behandlung mit einem Detergens alleine bezüglich der Virusinaktivierung relativ unwirksam ist. Für eine effektive Behandlung wird in dieser Literatur ein Gemisch aus einem Detergens und einem Di- oder Trialkylphosphat vorgeschlagen. Hierbei betrug die Konzentration des Detergens 1 % und des Lösungsmittels 0,1 %.

Eine kombinierte Behandlung mit einem organischen Lösungsmittel/Detergens und mit Wärme, wobei das Blutprodukt in trockenem Zustand erhitzt wird, ist ebenfalls in der Literatur beschrieben (American Journal of Hematology 35, 142 (1990)).

Virusinaktivierungsverfahren werden heutzutage als wirksam bezeichnet, wenn nach Anwendung des Verfahrens an einer Blutprodukt-Probe, welche mit einer hohen Dosis eines Testvirus versetzt wurde (z.B. entsprechend einem maximal möglichen Titer von ca. 10⁵ in einer Gerinnungsfaktor-Präparation), keine Viren mehr in der Probe nachgewiesen werden können und der Virustiter somit unter die Nachweisgrenze reduziert wurde.

Als Maß für die Inaktivierung ist der sogenannte Reduktionsfaktor bekannt, der nach einer einmaligen Zugabe von Testvirus aus dem dekadischen Logarithmus des Quotienten von Anfangs- und Endvirustiter errechnet wird. Aus der Richtlinie EC III/8115/89-EN der Kommission der Europäischen Gemeinschaften ist weiters der sogenannte Gesamt-Reduktionsfaktor bekannt. Er wird aus der Summe der Reduktionsfaktoren von einzelnen subsequenten Inaktivierungsmaßnahmen berechnet.

In der modernen Medizin besteht die Notwendigkeit, viele Blutprodukte über lange Zeit - in vielen Fällen auch als Dauerbehandlung - in großen Mengen, u.zw. auch zur Prophylaxe, zu verabreichen. Dies führt zwangsläufig zu einer Kumulation infektiöser Partikel und somit zu einem wesentlich erhöhten Infektionsrisiko, selbst bei Verabreichung von bereits virusinaktivierten Präparaten.

Reduktionsfaktoren müssen mit der sogenannten "worst case situation" für eine Viruskontamination des gesamten Plasmapools verglichen werden. Aus der Zeitschrift für Allgemeine Medizin 65, 429-433 (1989) ist bekannt, daß trotz Verarbeitung von getestetem und für HIV-negativ befundenem Plasma ein HIV-Gehalt von 10⁵ ID/ml (infektiöse Einheiten pro Milliliter) in Plasmaderivaten möglich ist. Unter der Annahme, daß einem Patienten während seines Lebens insgesamt etwa 100 l eines Faktor VIII-Präparates verabreicht werden, muß daher ein Verfahren zur Virus-Inaktivierung von Plasmaderivaten eine Virustiterreduktion von mindestens 10¹⁰ erlauben, um eine Infektion eines Patienten mit AIDS-Viren zu vermeiden.

Die Erfindung stellt sich zur Aufgabe, ein verfahren zur Herstellung eines virussicheren Blutprodukts, ausgenommen Albumin, zur Verfügung zu stellen, von dem sowohl erwartet werden kann, daß die Übertragung von infektiösen Agentien selbst bei Verabreichung von großen Mengen an Blutprodukt ausgeschlossen ist, und welches trotzdem noch eine hohe biologische Aktivität besitzt.

Diese Aufgabe wird erfindungsgemäß gelöst durch ein Verfahren zur Herstellung eines gegenüber infektiösen Agentien inaktivierten Blutprodukts, ausgenommen Albumin, mit einem Gesamt-Virus-Reduktionsfaktor von mindestens 40, bei welchem die biologische Aktivität zu mindestens 50%, bezogen auf die Aktivität vor Durchführung der Inaktivierung der infektiösen Agentien, erhalten bleibt, wobei
a) das Blutprodukt in wässeriger Lösung, die mindestens 2%, vorzugsweise mindestens 5%, Detergens enthält, behandelt und anschließend in festem Zustand erhitzt wird, oder
b) das Blutprodukt in festem Zustand erhitzt und anschließend in einer wässerigen Lösung, die mindestens 2%, vorzugsweise 5%, Detergens enthält, behandelt wird.

Vorzugsweise besteht die Erfindung in einem Verfahren zur Herstellung eines gegenüber infektiösen Agentien inaktivierten Blutprodukts, ausgenommen Albumin, wobei das Blutprodukt in wässeriger Lösung, die mehr als 10 % Detergens enthält, behandelt und gegebenenfalls im festen Zustand erhitzt wird.

Eine andere Ausführungsform besteht in einem Verfahren zur Herstellung eines gegenüber infektiösen Agentien inaktivierten Blutprodukts, ausgenommen Albumin, mit einem Gesamt-Virus-Reduktionsfaktor von mindestens 40, mit einer biologischen Aktivität von mindestens 50 %, bezogen auf die Aktivität vor Durchführung der Inaktivierung der infektiösen Agentien, welches durch eine Inaktivierungsbehandlung, die zwei oder mehrere unterschiedliche Inaktivierungsmethoden umfaßt, wobei mindestens eine Methode aus einer Hitzebehandlung des Blutproduktes in festem und feuchtem Zustand mit einem Wassergehalt von 0,05 bis 0,70 besteht und mindestens eine Methode aus der Behandlung des Blutprodukts mit einer mindestens 2%igen, vorzugsweise mindestens 5%igen, wässerigen Detergenslösung besteht.

Die biologische Aktivität wird als enzymatische Aktivität (z.B. von Blutgerinnungsenzymen und Co-Faktoren), als Avidität (von Immunglobulinen) oder als antigene Aktivität - eventuell durch die Verwendung von Aktivitäts- bzw. Antigenmarker - bestimmt.

Das erfindungsgemäße Blutprodukt kann aus herkömmlichen Blutprodukten hergestellt werden, indem die Inaktivierungsbehandlung während einer Zeitdauer durchgeführt wird, welche ausreicht, um einen Gesamt-Virus-Reduktionsfaktor von mindestens 40 zu erzielen. Diese Zeitdauer kann experimentell an einer Blutprodukt-Probe ermittelt werden, indem während der Behandlung bestimmte Mengen Testvirus wiederholt zugesetzt werden, wobei jede Wiederholung erst dann vorgenommen wird, wenn der Virustiter auf einen bestimmten Wert, vorzugsweise unter die Nachweisgrenze, abgesunken ist. Der Gesamt-Virus-Reduktionsfaktor ergibt sich aus der Summe der einzelnen Reduktionsfaktoren.

Wenn also während einer Behandlung zur Virusinaktivierung Testvirus in ausgewählten zeitlichen Intervallen wiederholt einem biologischen Produkt zugegeben wird, können nach Bestimmung des Anfangs- und Endvirustiters der dekadische Logarithmus des Virustiter-Verhältnisses mit der Anzahl der Intervalle multipliziert und die Reduktionsfaktoren zu einem Gesamt-Virus-Reduktionsfaktor addiert werden. Diese Berechnung setzt voraus, daß die Virustiterreduktion nach der letzten Testvirusgabe nicht größer als die vorhergehenden Titerreduktionen ist, was sich auch gezeigt hat.

Als Test-Virus kann z.B. das AIDS-Virus oder das Sindbis-Virus (als Modellvirus für Hepatitis-Viren) herangezogen werden.

Die Erfindung beruht auf der Erkenntnis, daß eine Behandlung mit Tween oder Detergens nach dem Stand der Technik, welche bei Detergens-Konzentrationen unter 10 % vorgenommen wird, zu keinem befriedigenden Ergebnis führt, wenn das Blutprodukt keiner weiteren Methode zur Virusinaktivierung unterzogen wird. Dies kann auf einen Schutzeffekt von Proteinen für Viren gegen inaktivierende Agentien, wie etwa Detergentien, zurückgeführt werden. Dieser Schutzeffekt kann aber durch eine höhere Tween- bzw. Detergenskonzentration beseitigt werden, ohne die biologische Aktivität der Proteine wesentlich zu beeinträchtigen. Eine solche Vorgangsweise ermöglicht es, auf den Zusatz weiterer Substanzen, wie z.B. Lösungsmittel, deren toxische Wirkung bekannt ist, zu verzichten.

Es hat sich als vorteilhaft erwiesen, wenn die Behandlung mit Tween bzw. dem Detergens bei einer Konzentration von mehr als 10 % und weniger als 25 %Masse, während einer Zeitdauer zwischen 1 min und 30 min, insbesondere bei einem pH-Wert zwischen 5,5 und 8, bei Temperaturen zwischen 0°C und 56°C, vorteilhafterweise zwischen 15°C und 37°C und gegebenenfalls bei einer elektrischen Leitfähigkeit von 7 bis 20 mS, durchgeführt wird.

Ein bevorzugtes Verfahren zur Herstellung erfindungsgemäßer inaktivierter Blutprodukte besteht darin, daß vor oder nach der Behandlung mit der wässerigen Detergenslösung das Blutprodukt mit heißem Dampf behandelt wird, wobei das Blutprodukt in festem Zustand auf einen Gehalt an Wasser, Methanol oder Äthanol von mehr als 0,05 (5 %Masse) und weniger als 0,70 (70 %Masse), vorzugsweise weniger als 0,40 (40 %Masse) eingestellt und in einem geschlossenen Behälter bei einer Temperatur im Bereich von 50 bis 121°C behandelt wird.

Die Erfindung betrifft auch ein Verfahren zur Bestimmung der Virus-Inaktivierungskapazität einer Inaktivierungsbehandlung, welche mindestens eine Inaktivierungsmethode umfaßt, indem mittels eines Testvirus der Reduktionsfaktor bestimmt wird, welches dadurch gekennzeichnet ist, daß das Testvirus wiederholt während der mindestens einen Inaktivierungsmethode zugegeben wird und die einzelnen Reduktionsfaktoren der mindestens einen Inaktivierungsmethode gegebenenfalls mit den Reduktionsfaktoren weiterer Inaktivierungsmethoden zu einem Gesamt-Virus-Reduktionsfaktor addiert werden.

Durch die nachfolgenden Beispiele wird die Erfindung noch näher erläutert.

### Beispiel 1

Aus Humanplasma wurde eine Kryopräzipitatlösung enthaltend Gerinnungsfaktor VIII nach einem Verfahren, beschrieben in der AT-B 391 808, hergestellt. Die Lösung wurde auf 8 % Tween 80 eingestellt und mit einer HIV-1 Virussuspension 7 mal in 2 min Intervallen versetzt. Nach einer Gesamtinkubationsdauer von 14 min bei 25°C wurde das Virus zentrifugiert und der Titer bestimmt. Der Kontrollwert des Ansatzes ohne Tween-Zusatz betrug 10^{5,1}. Der Virustiter nach der Tween-Behandlung lag unter der Nachweisgrenze von 10^{0,5} und konnte aufgrund des negativen Tests der Reversen Transkriptase mit 0 beziffert werden. Das ergibt einen Virus-Reduktionsfaktor von 7 x 5,1 = 35,7.

Die von Tween befreite Lösung wurde wieder mit HIV-1 Virussuspension versetzt, lyophilisiert und nach dem Verfahren der EP-A-0 159 311 mit einem Wassergehalt von 8 % 10 h bei 60°C erhitzt. Der Virustiter wurde von 10^{6,2} auf 0 herabgesetzt.

Beide Inaktivierungsstufen ergaben somit einen Gesamt-Virus-Reduktionsfaktor von 41,9. Somit ist nachgewiesen, daß eine Faktor VIII-Präparation, welche unter den oben angegebenen Bedingungen der Detergens- und der Hitzebehandlung unterzogen wird, einem Gesamt-Virus-Reduktionsfaktor von 41,9 entspricht und als virussicher anzusehen ist.

Die Bestimmung der Restaktivität von Faktor VIII erfolgte mit Hilfe des Thromboplastin-Bildungstest (2-Stufen-Test).

Die Faktor VIII-Restaktivität wurde berechnet durch Bildung des Quotienten aus der Faktor VIII-Aktivität der erhitzten Probe und der Faktor VIII-Aktivität des Ausgangsmaterials vor der Tween-Behandlung und betrug 80 %.

### Beispiel 2

Eine die Gerinnungsfaktoren II, IX und X enthaltende Präparation (Partieller Prothrombinkomplex, PPK) wurde nach der in Vox. Sang. 33, 37-50 (1977) beschriebenen Methode aus menschlichem Plasma durch Adsorption an DEAE-Sephadex, Waschen des Ionenaustauschers und Elution des Komplexes gewonnen.

Der PPK wurde in einer 22 % Tween 80-hältigen Lösung mit HIV-1 Virus versetzt und bei 25°C inkubiert. Die Virussuspension wurde 15 mal in 20 s Intervallen zugegeben. Der Virustiter der Kontrolle ohne Tween-Zusatz belief sich auf 10^{5,7}. Der Endvirustiter lag nach der Tween-Behandlung unter der Nachweisgrenze von 10^{0,5}. Daraus errechnet sich ein Gesamt-Virus-Reduktionsfaktor von mindestens 15 x 5,2 = 78. Eine PPK-Präparation, welche der obigen Tween-Behandlung unterzogen wird, entspricht somit einem Gesamt-Virus-Reduktionsfaktor von 78 und ist als virussicher anzusehen.

Die Aktivität der Gerinnungsfaktoren wurde am Beispiel des Faktor IX über den Zusatz der zu testenden Probe zu einem Faktor IX-Mangelplasma und Bestimmung der aktivierten partiellen Thromboplastinzeit (1-Stufen-Test) bestimmt und wurde durch die Behandlung mit Tween kaum beeinflußt. Das Verhältnis der Aktivität der behandelten Probe zur Aktivität des Faktor IX im unbehandelten PPK lag annähernd bei 100 %.

### Beispiel 3

Eine in Beispiel 2 beschriebene PPK-Präparation wurde mit 12 % Dimethyloctylamin-N-oxid in Gegenwart von Modellviren (Sindbis bzw. Vesikuläres Stomatitis Virus=VSV) bei 25°C inkubiert. Der Zusatz von Virussuspension erfolgte 10 mal in 5 min Intervallen. Nach der Behandlung mit Detergens lag der Virustiter jeweils unter der Nachweisgrenze von 10^{1,5}. Die Kontrollwerte ohne Detergenszusatz lagen bei 10^{6,4} bzw. 10^{6,1}. Daraus errechnet sich ein Gesamt-Virus-Reduktionsfaktor von mindestens 10 x 4,9 = 49 bzw. 10 x 4,6 = 46.

Die biologische Aktivität wurde durch die Detergensbehandlung kaum beeinträchtigt und betrug annähernd 100 %.

### Beispiel 4

Plasma wurde nach Cohn fraktioniert, und die COHN I-Fraktion enthaltend Fibrinogen mit Modellviren (Sindbis bzw. VSV) versetzt. Nach der Lyophilisation wurde das Konzentrat mit einem Wassergehalt von 8 % nach dem Verfahren der EP-A-0 159 311 10 h bei 60°C und anschließend 3 h bei 80°C erhitzt. Der Virustiter wurde von 10^{5,5} bzw. 10⁶ durch die Lyophilisation auf 10^{4,9} bzw. 10^{5,5} und weiters durch die Behandlung bei 60°C auf unter die Nachweisgrenze von 10^{0,5} gesenkt.

Die Inaktivierungskapazität der zweiten Behandlungsstufe bei 80°C wurde im Parallelansatz bestimmt: Durch die Lyophilisation wurde der Virustiter wieder von 10^{5,5} bzw. 10^{6,0} auf 10^{4,9} bzw. 10^{5,5} reduziert, und in weiterer Folge durch die Behandlung bei 80°C unter die Nachweisgrenze.

Der Reduktionsfaktor errechnet sich aus der zweimaligen Reduktion um 5 bzw. 5,5 Log-Stufen minus 0,6 bzw. 0,5 Log-Stufen, da die Fibrinogen-Präparation während des zweistufigen Behandlungsverfahrens nur einer einzigen Lyophilisation unterworfen wurde. Der Reduktionsfaktor betrug also 9,4 bzw. 10,5.

Das Pulver wurde anschließend in einem 5 % Octylglucosid-hältigen Medium gelöst und in Intervallen von 5 min 9 mal mit Sindbis bzw. VSV versetzt. Nach der Inkubation (insgesamt 45 min bei 25°C) wurde der Virustiter bestimmt. Die Behandlung mit Detergens reduzierte den Virustiter auf einen Wert unter der Nachweisgrenze von 10^{0,5}. Der Kontrollwert eines Ansatzes ohne Detergenszugabe betrug 10^{6,9} bzw 10^{6,0}. Daraus errechnet sich ein Virus-Reduktionsfaktor von mindestens 57,6 bzw. 49,5. Der Gesamt-Virus-Reduktionsfaktor betrug somit mindestens 67,0 bzw. 60,0. Eine Fibrinogen-Präparation, welche unter den oben angegebenen Bedingungen der Hitze- und Detergensbehandlung unterzogen wird, entspricht einem Gesamt-Virus-Reduktionsfaktor von mindestens 60,0 und ist als virussicher anzusehen.

Die biologische Aktivität des Fibrinogens wurde in dem mit 8 % Äthylalkohol gefällten Niederschlag aus der Octylglucosid-hältigen Fraktion mit einem Vernetzungstest der Fibrin-α-Ketten (T. Seelich, H.Redl "Theoretische Grundlagen des Fibrinklebers" in K. Schimpf "Fibrinogen, Fibrin und Fibrinkleber", F.K. Schattauer Verlag, Stuttgart-New York, 199-208, 1980) und mittels Thrombelastographie (H. Hartert in "Thrombosis and Bleeding Disorders", (N.U. Bang et al., eds.) Georg Thieme Verlag Stuttgart, Acad. Press New York London, 70-76, 1971) jeweils mit Zusatz von Gerinnungsfaktor XIII, bestimmt.

Die biologische Aktivität des behandelten Fibrinogens, bezogen auf die biologische Aktivität der COHN I-Fraktion, betrug 87 % gemessen an der Vernetzung und 56 % gemessen im Thrombelastogramm.

### Beispiel 5

Selektiertes Plasma wurde nach Cohn fraktioniert. Die COHN III-Fraktion, enthaltend anti-Tetanus-Toxoid-Gamma-Globulin wurde in Gegenwart von 15 % Triton X-100 mit HIV-1 bzw. Sindbis-Virus versetzt und bei 25°C inkubiert. Die Viruszugabe erfolgte 30 mal in Minutenabständen. Nach der Inkubationzeit von insgesamt 30 min lag der Virustiter unter der Nachweisgrenze von 10^{2,5} bzw. 10^{1,5}. Der Kontrollwert des Ansatzes ohne Detergenszugabe belief sich auf 10^{5,7} bzw. 10^{7,5}. Daraus errechnet sich ein Gesamt-Virus-Reduktionsfaktor von mindestens 30 x 3,2 = 96 bzw. 30 x 6 = 180. Eine Gamma-Globulin-Präparation, welche der obigen Detergensbehandlung unterzogen wird, entspricht einem Gesamt-Virus-Reduktionsfaktor von mindestens 96 und ist als virussicher anzusehen.

Die biologische Aktivität wurde mit einem Aviditäts-Test bestimmt. Dazu wurde Tetanus-Toxoid auf einer Mikrotiterplatte adsorbiert, mit Gelatine abgedeckt und gewaschen. Anschließend wurde das zu testende Gamma-Globulin in mehreren Verdünnungen auf die präparierte Mikrotiterplatte aufgetragen und nicht adsorbiertes Immunglobulin weggewaschen. Das an das Tetanus-Toxoid gebundene Gamma-Globulin wurde durch Adsorption eines anti human-IgG-Peroxidase Konjugats an den Fc-Teil des Immunglobulins und weiters durch die Farbreaktion der Peroxidase mit Diaminobenzidin und H₂O₂ und anschließende Extinktionsmessung bestimmt.

Die Avidität des Gamma-Globulins für das Tetanus-Toxoid wurde durch die Behandlung mit Detergens kaum beeinflußt. Es wurden keine signifikanten Aviditäts-Unterschiede vor und nach der Behandlung beobachtet.

### Beispiel 6

0,95 ml einer Lösung enthaltend C₁-Esterase-Inhibitor (hergestellt nach Vogelaar E F et al (1973) Vox Sang. 26, 118-127 "Contributions to the Optimal Use of Human Blood") wurden mit 20 mg Triton X-100 versetzt und bei 25°C inkubiert. Zur Bestimmung der Virusinaktivierungskapazität wurde VSV-Virus (10 µl) wiederholt im Abstand von 5 min zugesetzt. Nach 5-maligem Viruszusatz lag der Virustiter unter der Nachweisgrenze von 10^{0,5}. Der Kontrollwert des Ansatzes ohne Detergenszugabe belief sich auf 10^{7,5}. Daraus errechnet sich ein Virus-Reduktionsfaktor von mindestens 5 x 7 = 35.

Der C₁-Esterase-Inhibitor wurde an DEAE-Sephadex adsorbiert und mit 8,89 g/l NaCl-Lösung solange gewaschen, bis er frei von Detergens war. Nach Desorption des Inhibitors mit 59 g/l NaCl-Lösung wurde gegen einen Puffer enthaltend 1,0 g/l Natriumcitrat und 0,4 g/l NaCl (pH 6,8) dialysiert. Dieser Lösung wurde erneut VSV-Virus zugesetzt, bevor sie lyophilisiert wurde. Das Präparat wurde 24 h lang bei 72°C trocken erhitzt. Während der Lyophilisierung und anschließenden Hitzebehandlung wurde der Virustiter von 10^{7,2} auf unter die Nachweisgrenze von 10^{0,5} reduziert. Der Virus-Reduktionsfaktor betrug demnach mindestens 6,7.

Aus den Virus-Reduktionsfaktoren der Detergens- und der Hitzebehandlung errechnet sich ein Gesamt-Virus-Reduktionsfaktor von mindestens 41,7. Die biologische Aktivität des Inhibitors wurde durch die Inaktivierung der VSV-Viren kaum beeinträchtigt und betrug annähernd 100 %.

## Patentansprüche

1. Verfahren zur Herstellung eines gegenüber infektiösen Agentien inaktivierten Blutprodukts, ausgenommen Albumin, mit einem Gesamt-Virus-Reduktionsfaktor von mindestens 40, bei welchem die biologische Aktivität zu mindestens 50%, bezogen auf die Aktivität vor Durchführung der Inaktivierung der infektiösen Agentien, erhalten bleibt, wobei
a) das Blutprodukt in wässeriger Lösung, die mindestens 2%, vorzugsweise mindestens 5%, Detergens enthält, behandelt und anschließend in festem Zustand erhitzt wird, oder
b) das Blutprodukt in festem Zustand erhitzt und anschließend in einer wässerigen Lösung, die mindestens 2%, vorzugsweise 5%, Detergens enthält, behandelt wird.

2. Verfahren zur Herstellung eines gegenüber infektiösen Agentien inaktivierten Blutprodukts, ausgenommen Albumin, mit einem Gesamt-Virus-Reduktionsfaktor von mindestens 40, bei welchem die biologische Aktivität zu mindestens 50%, bezogen auf die Aktivität vor Durchführung der Inaktivierung der infektiösen Agentien, erhalten bleibt, wobei das Blutprodukt in wässeriger Lösung, die mehr als 10% Detergens enthält, behandelt und gegebenenfalls im festen Zustand erhitzt wird.

3. Verfahren zur Herstellung eines gegenüber infektiösen Agentien inaktivierten Blutprodukts, ausgenommen Albumin, mit einem Gesamt-Virus-Reduktionsfaktor von mindestens 40, bei welchem die biologische Aktivität zu mindestens 50%, bezogen auf die Aktivität vor Durchführung der Inaktivierung der infektiösen Agentien, erhalten bleibt, wobei das Inaktivierungsverfahren zwei oder mehrere unterschiedliche Inaktivierungsmethoden umfaßt und mindestens eine Methode aus einer Hitzebehandlung des Blutprodukts im festen und feuchten Zustand mit einem Wassergehalt von 0,05 bis 0,70 besteht und mindestens eine Methode aus der Behandlung des Blutprodukts mit einer mindestens 2%-igen, vorzugsweise mindestens 5%-igen, wässerigen Detergenslösung besteht.

4. Verfahren nach einem der Ansprüche 1 bis 3, dadurch gekennzeichnet, daß das Blutprodukt mit heißem Dampf behandelt wird, wobei das Blutprodukt in festem Zustand auf einen Gehalt an Wasser, Methanol oder Äthanol von mehr als 0,05 (5% Masse) und weniger als 0,70 (70% Masse), vorzugsweise weniger als 0,40 (40% Masse) eingestellt und in einem geschlossenen Behälter bei einer Temperatur im Bereich von 50 bis 121°C behandelt wird.

5. Verfahren zur Bestimmung der Virus-Inaktivierungskapazität einer Inaktivierungsbehandlung, welches mindestens eine Inaktivierungsmethode umfaßt, indem mittels eines Testvirus der Reduktionsfaktor bestimmt wird, dadurch gekennzeichnet, daß das Testvirus wiederholt während der mindestens einen Inaktivierungsmethode zugegeben wird und die einzelnen Reduktionsfaktoren der mindestens einen Inaktivierungsmethode gegebenenfalls mit den Reduktionsfaktoren weiterer Inaktivierungsmethoden zu einem Gesamt-Virus-Reduktionsfaktor addiert werden.

## Claims

1. A method of producing a blood product, exclusive of albumin, inactivated relative to infectious agents, having a total virus reduction factor of at least 40, in which the biologic activity is maintained by at least 50%, based on the activity prior to effecting inactivation of the infectious agents, wherein
a) the blood product is treated in an aqueous solution containing at least 2%, preferably at least 5%, of a detergent, and subsequently is heated in the solid state, or
b) the blood product is heated in the solid state and subsequently is treated in an aqueous solution containing at least 2%, preferably 5%, of a detergent.

2. A method of producing a blood product, exclusive of albumin, inactivated relative to infectious agents, having a total virus reduction factor of at least 40, in which the biologic activity is maintained by at least 50%, based on the activity prior to effecting inactivation of the infectious agents, wherein the blood product is treated in an aqueous solution containing more than 10% of a detergent and optionally is heated in the solid state.

3. A method of producing a blood product, exclusive of albumin, inactivated relative to infectious agents, having a total virus reduction factor of at least 40, in which the biologic activity is maintained by at least 50%, based on the activity prior to effecting inactivation of the infectious agents, wherein the inactivation process comprises two or more different inactivation methods and at least one method consists in a heat treatment of the blood product in the solid and wet state having a water content of from 0.05 to 0.70, and at least one method consists in the treatment of the blood product with an at least 2%, preferably at least 5%, aqueous detergent solution.

4. A method according to any one of claims 1 to 3, characterised in that the blood product is treated with hot vapor, wherein the blood product in the solid state is adjusted to a content of water, methanol or ethanol of more than 0.05 (5% by mass) and less than 0.70 (70% by mass), preferably less than 0.40 (40% by mass) and treated in a closed container at a temperature in the range of from 50 to 121°C.

5. A method of determining the virus inactivation capacity of an inactivation treatment which comprises at least one inactivation method, wherein the reduction factor is determined by means of a test virus, characterised in that the test virus is repeatedly admixed during the at least one inactivation method and the individual reduction factors of the at least one inactivation method optionally are added up with the reduction factors of further inactivation methods to give a total virus reduction factor.

## Revendications

1. Procédé de préparation d'un produit sanguin, à l'exception de l'albumine, inactivé en ce qui concerne les agents infectieux, avec un facteur de réduction totale des vies d'au moins 40, l'activité biologique étant conservée à au moins 50 % par rapport à l'activité avant la mise en oeuvre de l'inactivation des agents infectieux, dans lequel
c) on traite le produit sanguin dans une solution aqueuse qui contient au moins 2 %, de préférence au moins 5 %, de détergent, puis on le chauffe à l'état solide, ou
d) on chauffe le produit sanguin à l'état solide, puis on le traite dans une solution aqueuse qui contient au moins 2 %, de préférence au moins 5 %, de détergent.

2. Procédé de préparation d'un produit sanguin, à l'exception de l'albumine, inactivé en ce qui concerne les agents infectieux, avec un facteur de réduction totale des virus d'au moins 40, l'activité biologique étant conservée à au moins 50 % par rapport à l'activité avant la mise en oeuvre de l'inactivation des agents infectieux, dans lequel on traite le produit sanguin dans une solution aqueuse contenant plus de 10 % de détergent, et éventuellement on le chauffe à l'état solide.

3. Procédé de préparation d'un produit sanguin, à l'exception de l'albumine, inactivé en ce qui concerne les agents infectieux, avec un facteur de réduction totale des virus d'au moins 40, l'activité biologique étant conservée à au moins 50 % par rapport à l'activité avant la mise en oeuvre de l'inactivation des agents infectieux, dans lequel le traitement d'inactivation comprend deux ou plusieurs méthodes d'inactivation différentes, au moins une méthode consistant en un traitement thermique du produit sanguin à l'état solide et humide avec une teneur en eau de 0,05 à 0,70, et au moins une méthode consistant en un traitement du produit sanguin avec une solution aqueuse de détergent à au moins 2%, de préférence au moins 5 %.

4. Procédé selon l'une des revendications 1 à 3, caractérisé en ce que l'on traite le produit sanguin avec de la vapeur chaude, en réglant la teneur en eau, en méthanol ou en éthanol du produit sanguin à l'état solide à plus de 0,05 (5 % en masse) et à moins de 0,70 (70 % en masse), de préférence à moins de 0,40 (40 % en masse) et en effectuant le traitement dans un récipient fermé à une température comprise entre 50 et 121°C.

5. Procédé de détermination de la capacité d'inactivation des virus d'un traitement d'inactivation comprenant au moins une méthode d'inactivation, par détermination du facteur de réduction à l'aide d'un virus test, caractérisé en ce que l'on effectue des additions répétées du virus test pendant au moins cette méthode d'inactivation, et on additionne éventuellement les différents facteurs de réduction d'au moins cette méthode d'inactivation avec les facteurs de réduction d'autres méthodes d'inactivation pour obtenir un facteur de réduction totale des virus.
